(19)

**Europäisches Patentamt**

**European Patent Office**

**Office européen des brevets**

(11) **EP 0 970 938 A1**

(12) **EUROPEAN PATENT APPLICATION**

(43) Date of publication:
**12.01.2000 Bulletin 2000/02**

(51) Int. Cl.[7]: **C07C 17/18**, C07C 25/13, C07C 22/08, C07C 205/11

(21) Application number: **99113563.3**

(22) Date of filing: **07.07.1999**

(84) Designated Contracting States:
**AT BE CH CY DE DK ES FI FR GB GR IE IT LI LU MC NL PT SE**
Designated Extension States:
**AL LT LV MK RO SI**

(30) Priority: **08.07.1998 US 111674**

(71) Applicant:
**AIR PRODUCTS AND CHEMICALS, INC.**
**Allentown, PA 18195-1501 (US)**

(72) Inventors:
- **Casteel, William Jack Jr.**
  **Emmaus, PA 18049 (US)**
- **Pesaresi, Reno Joseph Jr.**
  **Easton, PA 18042 (US)**
- **Lal, Gauri Sankar**
  **Whitehall, PA 18052 (US)**

(74) Representative:
**Schwabe - Sandmair - Marx**
**Stuntzstrasse 16**
**81677 München (DE)**

(54) **Preparation of 1,4-bis-(difluoromethyl)benzene**

(57) Sulfur tetrafluoride is used at subambient temperatures and near ambient pressures to deoxofluorinate 1,4-terephthalaldehyde in a solvent of hydrogen fluoride and produce 1,4-bis-(difluoromethyl) benzene in very high yield and purity. This product is a precursor of octafluoro-[2,2]paracyclophane, known as "AF4" that can be polymerized into useful films having excellent dielectric properties. The HF can be diluted with neutral organic solvents.

EP 0 970 938 A1

## Description

CROSS-REFERENCE TO RELATED APPLICATIONS

Not applicable.

STATEMENT REGARDING FEDERALLY SPONSORED RESEARCH OR DEVELOPMENT

Not applicable.

BACKGROUND OF THE INVENTION

This invention relates to the preparation of 1,4-bis-(difluoromethyl)-2-X-benzene (where X = H, methyl, chloro, bromo, or nitro) by the reaction of the 2-X substituted 1,4-terepthaldehyde with sulfur tetrafluoride. In another aspect it relates to a process for carrying out such a reaction at ambient or near ambient pressures. In still another aspect it relates to a method of reducing the required pressure for this reaction by the use of hydrogen fluoride and subambient temperatures.

The compound 1,4-bis-(difluoromethyl)benzene (TFPX) is an important precursor in the preparation of octafluoro-(2,2)-paracyclophane (AF4) which in turn can be polymerized to form protective coatings that are very effective dielectrics. The formation of such coatings, which are called parylene, is described in U.S. Patent No. 5,210,341 to Dolbier, Jr. et al., issued May 11, 1993. As described in this patent, dibromo-tetrafluoro-p-xylene is dimerized using a titanium compound as a reducing agent to form AF4. Dibromo-tetrafluoro-p-xylene is made by brominating TFPX, as disclosed by Fuqua et al., *Tetrahedron*, 20, 1625, (1964). These authors describe treating 1,4-terephthalaldehyde with $SF_4$ at 150°C and 1300 psi to form 1,4-bis-(difluoromethyl)benzene, which was then brominated using $Br_2$ in $CCl_4$ with UV irradiation. There have been essentially no significant improvements from 1964 to the present in the formation of TFPX and the high pressures involved have been a deterrent to potential commercialization of this process.

Although there have been some advances in the use of $SF_4$ in the fluorination of carboxylic acids and esters and aliphatic ketones, these reactions are highly unpredictable and attempts to enhance yields or process conditions have at times resulted in altering the product obtained. Dmowski in *J. Fluor. Chem*., 32, 255, (1986) discusses the use of $SF_4$ as a fluorinating agent in reactions with carboxylic acids, aldehydes, ethers and amides and covers specifically the role of HF produced in the reaction as a catalyst with respect to fluorination of carboxylic acids. This author concluded that catalytic amounts of HF activate $SF_4$ by complexing with it, but, on the negative side, HF engages in a competing reaction with the carbonyl function to inhibit its reaction with the $SF_4$. Other authors report some enhancement of the fluorination of certain organic acids with the use of HF. A review of uses for $SF_4$ reported since 1959 is presented by Burmakov at al. in *New Uses of Sulfur Tetrafluoride in Organic Synthesis*, in "New Fluorinating Agents in Organic Synthesis", German and Zemskov, Eds., Springer-Verlag, p.198, (1989). Instances in which the efficiency of $SF_4$ was enhanced by the presence of HF were in reactions with sterically hindered aromatic and heterocyclic carboxylic acids, esters of carboxylic acids, and aliphatic ketones. Although yields were increased, in many cases the course of the reaction was altered, in some instances producing substitution of hydrogen in the molecule by fluorine. Hudlicky, in "Chemistry of Organic Fluorine Compounds", 2, 236-253, (1995) presents an exhaustive review of the literature on reactions in which $SF_4$ is used to replace carbonyl oxygen in aldehydes, ketones and carboxylic acids with fluorine. Reactions of some aromatic aldehydes are said to react cleanly in the presence of KF which serves as a hydrogen fluoride scavenger. On the other hand, reactions of alpha-keto acids are said to give trifluoro derivatives in high yields in the presence of anhydrous HF. A mechanism for the reaction of $SF_4$ and HF with alkane carboxylic acids is proposed.

The use of $SF_4$ for fluorinating organic compounds has been known for almost four decades, yet there have been little or no real improvements in the process for using this chemical to fluorinate aromatic aldehydes. Although HF has been observed to enhance yields in the fluorination of some carboxylic acids, esters and ketones, the avoidance of HF through the use of a scavenger has been noted to be of advantage with respect to aromatic aldehydes. Thus it appears that this area of organic synthesis is a highly empirical art where experimental evidence is required to reach useful conclusions.

BRIEF SUMMARY OF THE INVENTION

We have now found that 1,4-terephthalaldehyde can be deoxyfluorinated to form 1,4-bis-(difluoromethyl)benzene (TFPX) by reaction with $SF_4$ in solvent quantities of HF at subambient temperatures. As a result, this synthesis can be run at near ambient pressures, thereby greatly enhancing the safety and economics of potential commercial processes. Surprisingly, even at low temperatures of operation, the reaction proceeds in a reasonable time to form the desired product in high yields and purity. Similarly, 1,4-terepthaldehydes substituted at the 2 position with either electron donating (methyl) or electron withdrawing (chloro, bromo, nitro) groups can be deoxofluorinated at equally mild conditions. This chemistry is in accord with our findings that benzaldehyde and the highly deactivated 4-nitrobenzaldehyde react under mild conditions to give difluoromethylbenzene and difluoromethyl-4-nitrobenzene, respectively. In sharp contrast, deactivated aromatic ketones such as benzophenone show only traces of fluorination with $SF_4$ in liquid HF at room temperature overnight. Similarly, benzoic acid is fluorinated to benzoyl fluoride in liquid HF with $SF_4$ at —78°C, but undergoes only very slow fluorination to benzotrifluoride at room temperature. In our process the HF can be diluted with neutral organic solvents, such as methylene chloride, but slightly basic solvents, such as acetonitrile, made $SF_4$ nonreactive with 1,4-terephthalaldehyde even at room temperature.

## DETAILED DESCRIPTION OF THE INVENTION

While it is known that 1,4-terephthalaldehyde can be deoxofluorinated with $SF_4$ to form a low-K dielectric precursor, 1,4-bis-(difluoromethyl)benzene, this process has required high temperatures and pressures on the order of 150°C and 800 psig and above. These conditions, particularly the high pressures, would necessitate considerable expense in scaling up the process for commercialization. The subsequent reactions toward an end use product are known, requiring bromination of the 1,4-bis-(difluoromethyl)benzene to form dibromo-tetrafluoro-p-xylene which can then be dimerized to produce a product known as AF4. The AF4 can be polymerized to form thin protective coatings that have excellent dielectric and barrier properties. Such coatings, which are inert and transparent, are suitable for use in the electronics, automotive and medical industries.

Our invention solves the problem of high pressures in the deoxyfluoronation of the 1,4-terephthalaldehydes by carrying out the reaction in HF solvent at subambient temperatures. By "subambient temperatures" we mean temperatures that do not exceed 30°C and are preferably subzero, for example in the range of -60 to 0°C, and more preferably between -30 and 0°C. The corresponding pressures for the reaction mixture with temperatures in this latter range and with HF as the undiluted solvent in the system are shown in Table 1.

Table 1

| T (°C) | P (psig) |
|---|---|
| -27 | 0.0 |
| -10 | 11.5 |
| 0 | 24 |

The amount of $SF_4$ in the reaction in relation to the aldehyde should be at least stoichiometric so that one equivalent of $SF_4$ is present for each carbonyl or aldehyde group according to the following equation:

$$O{=}CH\text{-}(C_6H_6)\text{-}HC{=}O + 2SF_4 \rightarrow F_2CH\text{-}(C_6H_6)\text{-}HCF_2 + 2SOF_2$$

The amount of HF employed should be sufficient to serve as a solvent for the aldehyde and the product. There is no advantage to be gained in using more HF than is required to place the reactants and product in solution. The HF can be diluted with a neutral organic solvent, such as methylene chloride, chloroform, $CFCl_3$, hexane, and the like, but to do so will increase the reaction pressure even though it does not significantly alter the reactivity of $SF_4$ with 1,4-terephthalaldehyde. Any material used as a diluent for the HF must have negligible Lewis basicity; and an acidic diluent would compete with the aldehyde for the $SF_4$. The only reason for making such a dilution of the HF would be to reduce costs, so this is a trade-off against the higher cost of operating with elevated pressures and would depend upon the particular circumstances under which the process is commercialized. The HF should be anhydrous to avoid side reactions and, of course, any diluent should also be devoid of water. The amount of diluent that can be tolerated depends upon the reaction time and temperature selected for the process, but in general the molar ratio of HF to $SF_4$ should not go below 1.0.

The process can be conducted in either a batch or continuous operation, although batch procedures are preferred to facilitate control of the temperature and pressure. The reactants should be refrigerated initially to cool them below ambient temperatures and it is preferred to start with the reactants considerably below 0°C and allow the reaction mixture to warm under agitation to the desired reaction temperature. The pressure of the reaction is readily controlled in this manner below 65 psig and preferably within a range of 0 to 25 psig.

To further illustrate our invention and a typical recovery process, the following Examples are presented which should be considered as demonstrative of the invention but not to limit it unduly.

EXAMPLE

Example 1

A 300 ml Parr reactor was charged with 40 grams (298 mmol) 1,4-terephthalaldehyde. Although a cosolvent was not used in this Example, if one were to be used it would also be charged to the reactor with the aldehyde. The Parr reactor was then cooled to ~ -55°C and 55 ml of anhydrous hydrogen fluoride was condensed in under static vacuum. While stirring this mixture at 200 rpm, 621 mmol of $SF_4$, equal to slightly more than one equivalent of $SF_4$ per aldehyde group in the 1,4-terephthalaldehyde, was transferred into the reactor at -55°C under static vacuum. The reaction mixture was then allowed to warm to 0°C. (This reaction has been observed to proceed rapidly between -30 and 20°C.) The $SF_4/SOF_2$ vapor pressures above the reaction solution were observed as shown in Table 1. Stirring the reaction mixture at 200 rpm was continued with the temperature at 0°C for 1.5 hours.

At this point the solution was cooled to -10°C and the volatiles were evacuated through a soda lime scrubber. When the reactor pressure remained below 1 Torr under a static vacuum, the reactor was removed from the system and opened. Fifty ml of water was added followed by bicarbonate to destroy any remaining HF. The product was extracted from the resulting mixture with $CH_2Cl_2$. Hexane can also be used for this purpose. In this run 52.36 grams of liquid product were isolated, amounting to a yield of 95 percent. Analysis of this product by gas chromatography and mass spectrometry and by NMR spectroscopy confirmed its identity as 1,4-bis-(difluoromethyl)-benzene (TFPX) at 99 percent purity. The only observable impurity was 4-(difluoromethyl)-benzoylfluoride present at less than 1 percent of the total product.

Example 2

A 50 ml FEP reactor with stainless steel fittings is charged with 2-methyl-1,4-terephthaldehyde. Enough anhydrous HF is distilled into the reactor at -50°C to completely dissolve the aldehyde at that temperature. The solution is magnetically stirred, and 2.2 equivalents of $SF_4$ are condensed into the solution at -50°C. As the stirred reaction mixture warms to 0°C, the $SF_4/SOF_2$ vapor pressure increases. After no further pressure increase is observed, the solution is cooled to -10°C, and the volatiles are evacuated through a soda-lime scrubber. When the reaction pressure remains constant under static vacuum, any remaining HF in the product slurry is neutralized with aqueous bicarbonate. The product is extracted with ether, although methylene chloride or hexane may also be used for this purpose. Analysis by gas chromatography and mass spectrometry are used to show that the product is 1,4-bis-(difluoromethyl)-2-methylbenzene.

Example 3

A 50 ml FEP reactor with stainless steel fittings is charged with 2-chloro-1,4-terephthaldehyde. Enough anhydrous HF is distilled into the reactor at -50°C to completely dissolve the aldehyde at that temperature. The solution is magnetically stirred, and 2.2 equivalents of $SF_4$ are condensed into the solution at -50°C. As the stirred reaction mixture warms to 0°C, the $SF_4/SOF_2$ vapor pressure increases. After no further pressure increase is observed, the solution is cooled to -10°C, and the volatiles are evacuated through a soda-lime scrubber. When the reaction pressure remains constant under static vacuum, any remaining HF in the product slurry is neutralized with aqueous bicarbonate. The product is extracted with ether, although methylene chloride or hexane may also be used for this purpose. Analysis by gas chromatography and mass spectrometry are used to show that the product is 1,4-bis-(difluoromethyl)-2-chlorobenzene.

Example 4

A 50 ml FEP reactor with stainless steel fittings is charged with 2-bromo-1,4-terephthaldehyde. Enough anhydrous HF is distilled into the reactor at -50°C to completely dissolve the aldehyde at that temperature. The solution is magnetically stirred, and 2.2 equivalents of $SF_4$ are condensed into the solution at -50°C. As the stirring reaction mixture warms to 0°C, the $SF_4/SOF_2$ vapor pressure increases. After no further pressure increase is observed, the solution is cooled to -10°C, and the volatiles are evacuated through a soda-lime scrubber. When the reaction pressure remains constant under static vacuum, any remaining HF in the product slurry is neutralized with aqueous bicarbonate. The product is extracted with ether, although methylene chloride or hexane may also be used for this purpose. Analysis by gas chromatography and mass spectrometry are used to show that the product is 1,4-bis-(difluoromethyl)-2-bromobenzene.

Example 5

A 50 ml FEP reactor with stainless steel fittings is charged with 2-nitro-1,4-terephthaldehyde. Enough anhydrous HF is distilled into the reactor at -50°C to completely dissolve the aldehyde at that temperature. The solution is magnetically stirred, and 2.2 equivalents of $SF_4$ are condensed into the solution at -50°C. As the stirring reaction mixture warms to 0°C, the $SF_4$/$SOF_2$ vapor pressure increases. After no further pressure increase is observed, the solution is cooled to -10°C, and the volatiles are evacuated through a soda-lime scrubber. When the reaction pressure remains constant under static vacuum, any remaining HF in the product slurry is neutralized with aqueous bicarbonate. The product is extracted with ether, although methylene chloride or hexane may also be used for this purpose. Analysis by gas chromatography and mass spectrometry are used to show that the product is 1,4-bis-(difluoromethyl)-2-nitrobenzene.

The products of these Examples can be used directly in further synthesis as described above and by procedures known in the art to form the material known as "AF4" which is convertible into useful protective films and dielectrics. Other advantages and features of our invention will be apparent to those skilled in the art from the foregoing disclosure and the following claims.

**Claims**

1. A process for making 1,4-bis-(difluoromethyl)-2-X-benzene wherein X is H, methyl, chloro, bromo, or nitro, which process comprises reacting 2-X substituted 1,4-terephthalaldehyde with $SF_4$ in the presence of sufficient HF to act as a solvent in the reaction at subambient temperature.

2. The process of Claim 1 wherein said temperature is in the range -60 to 30°C.

3. The process of Claim 1 wherein said $SF_4$ is used in at least a stoichiometric amount in relation to said 1,4-terephthalaldehyde.

4. The process of Claim 3 wherein said temperature is in the range of -30 to 0°C.

5. The process of Claim 1 wherein said HF is diluted with a neutral organic solvent.

6. The process of Claim 1 wherein the pressure is near ambient pressure and does not exceed 65 psig.

7. The process of Claim 6 wherein said pressure is in the range of 0 to 25 psig.

8. The process of Claim 5 wherein said neutral organic solvent is methylene chloride.

9. The process of Claim 3 wherein the amount of said $SF_4$ in relation to the 1,4-terephthalaldehyde is about one equivalent of $SF_4$ per aldehyde group.

10. The process of Claim 1 wherein 1,4-terephthalaldehyde is reacted with $SF_4$ to make 1,4-bis-(difluoromethyl)benzene.

11. A process for deoxofluorinating 2-X substituted 1,4-terephthalaldehyde whrein X is H, methyl, chloro, bromo, or nitro, with $SF_4$ which comprises:

    (a) forming a reaction mixture of said 1,4-terephthalaldehyde and a solvent amount of hydrogen fluoride under refrigerated conditions below ambient temperature,

    (b) adding to said mixture at least a stoichiometric amount of $SF_4$ while maintaining the temperature of the reaction mixture under said refrigerated conditions,

    (c) allowing said mixture of step (b) to warm to a temperature below ambient conditions so that the pressure of said process does not exceed 65 psig, and

    (d) recovering as a product of the process 1,4-bis-(difluoromethyl)-2-X-benzene.

12. The process of Claim 11 wherein each of said conditions and said temperature of step (c) include a temperature in the range of -60 to 0°C and a pressure below 25 psig.

**13.** The process of Claim 11 wherein said process is carried out at a temperature from -30°C to 0°C.

**14.** The process of Claim 11 wherein said HF of step (a) is present in at least equal molar amount in relation to said $SF_4$ of step (b).

European Patent Office

# EUROPEAN SEARCH REPORT

Application Number
EP 99 11 3563

## DOCUMENTS CONSIDERED TO BE RELEVANT

| Category | Citation of document with indication, where appropriate, of relevant passages | Relevant to claim | CLASSIFICATION OF THE APPLICATION |
|---|---|---|---|
| X | US 2 859 245 A (SMITH WILLIAM C) 4 November 1958 (1958-11-04) * column 2, line 2 - line 34; claims; example 8 * | 1-10 | C07C17/18 C07C25/13 C07C22/08 C07C205/11 |
| A | US 3 332 891 A (CHOW SUI-WU ET AL) 25 July 1967 (1967-07-25) * column 5, line 1 - line 8 * | 1 | |
| A | DOLBIER W R, JR. ET AL: "New synthesis of octafluoro‘2.2!paracyclophane" J. ORG. CHEM. (JOCEAH,00223263);1993; VOL.58 (7); PP.1827-30, XP002113138 Univ. Florida;Dep. Chem.; Gainesville; 32611-2046; FL; USA (US) * page 1829, column 1 * | 1 | |

TECHNICAL FIELDS SEARCHED

C07C

The present search report has been drawn up for all claims

| Place of search | Date of completion of the search | Examiner |
|---|---|---|
| THE HAGUE | 24 August 1999 | Bonnevalle, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 99 11 3563

This annex lists the patent family members relating to the patent documents cited in the above-mentioned European search report.
The members are as contained in the European Patent Office EDP file on
The European Patent Office is in no way liable for these particulars which are merely given for the purpose of information.

24-08-1999

| Patent document cited in search report | Publication date | Patent family member(s) | Publication date |
|---|---|---|---|
| US 2859245 A | 04-11-1958 | CH 371792 A | |
| | | DE 1080993 B | |
| | | FR 1225634 A | 01-07-1960 |
| | | GB 823336 A | |
| US 3332891 A | 25-07-1967 | FR 1414095 A | 05-01-1966 |
| | | FR 1414096 A | 05-01-1966 |
| | | GB 1086714 A | |
| | | NL 6411018 A | 24-03-1965 |
| | | NL 6411020 A | 24-03-1965 |
| | | NL 6411094 A | 24-03-1965 |
| | | US 3274267 A | 20-09-1966 |
| | | GB 1086712 A | |
| | | NL 6411019 A | 24-03-1965 |
| | | GB 1074708 A | |



For more details about this annex : see Official Journal of the European Patent Office, No. 12/82